# EUROPEAN PATENT APPLICATION

(11) **EP 2 407 144 A1**
(43) Date of publication of application: **18.01.2012**
(21) Application number: 11173048.7
(22) Date of filing: 07.07.2011
(51) Int. Cl.: A61K 8/02, A61K 8/04, A61K 8/81, B65D 83/38, B65D 83/14, A61Q 5/06

(54) **Aerosol hairspray product for styling and/or shaping hair**

(30) Priority: 13.07.2010 EP 10169383
(71) Applicant: The Procter & Gamble Company, Cincinnati, OH 45202 (US)
(72) Inventor: Birkel, Susanne, 64295 Darmstadt (DE); Batt, Marylou, Sharonville, OH Ohio 45241 (US)
(74) Representative: Marollé, Patrick Pierre Pascal

(57) **Abstract**

An aerosol hairspray product for styling and/or shaping hair. The product comprises a container for storing a hairstyling formulation and a propellant, wherein the container wall comprises at least about 80% plastic material by total weight of the container. The product also comprises a hairstyling formulation comprising from about 35% to about 99% water by total weight of the hairstyling formulation and propellant. Furthermore, the product comprises from 15% to 54% volatile organic compound by total weight of the hairstyling formulation and propellant.

## Description

### FIELD OF THE INVENTION

In a first aspect, an aerosol hairspray product for styling and/or shaping hair is provided. The product comprises a container for storing a hairstyling formulation and a propellant, wherein the container wall comprises at least about 80% plastic material by total weight of the container. The product also comprises a hairstyling formulation comprising from about 35% to about 99% water by total weight of the hairstyling formulation and propellant. Furthermore, the product comprises from 15% to 54% volatile organic compound by total weight of the hairstyling formulation and propellant. In a second aspect, a method for styling hair is provided comprising the steps of: applying to hair a composition ejected by the hairspray product; drying the ejected composition on the hair. In a third aspect, a use of the product for fixing and/or shaping a hairstyle is provided

### BACKGROUND OF THE INVENTION

Hairstyling products such as hairsprays are used for achieving different hairstyles and for holding hair strands in place for a period of time. Typically, hairsprays comprise film-forming polymers, which when applied to keratin-containing fibres, such as human hair, form fibre-fibre welds. These welds 'glue' the fibres together and hence impart hold to the hairstyle. Finishing hairsprays are used to spray on an already achieved hairstyle and left to dry without disturbing the hairstyle. Shaping hairsprays are typically applied to the hair before the final hairstyle has been achieved - the shaping hairspray is applied and the hair immediately styled before the composition dries on the hair.

Aerosol hairspray products usually comprise a pressure-resistant container, a nozzle, a propellant, and a hairstyling formulation. A hairspray composition is normally ejected from such products via a mechanical pump nozzle or aerosol-forming nozzle. See, for example, US2009/0104138A1. Commonly used propellants include the volatile organic compounds (VOCs) propane, butane, 1,1-difluoroethane, and dimethylether. However, VOCs are known to react with certain nitrogenic oxides, which in turn may result in the formation of ground-level ozone - a potential source of health problems. Alcohols are also often used in the hairstyling formulation, for example to reduce surface tension. However, a high proportion of alcohol may leave the hair feeling dry and brittle and some alcohols may cause an allergic response in some users. The container is normally made from metal which is mechanically strong, but metal is a non-renewable resource and expensive to recycle. Metal may also have the disadvantage that if the pressure inside the container is allowed to build up, once a critical pressure level is reached, the container may explode, which provides the potential to cause damage to surroundings due to flying shards of metal. The metal container is usually provided in a simple, cylindrical shape. Metal corrosion may also be a problem if the contained formulation is water-based - the inside of the container normally requires a coating to physically separate the formulation from the metal and thus prevent the corrosive oxidative reactions. Moreover, corrosion inhibitors may be needed in the hairspray formulation itself.

Altering one or more features of an aerosol hairspray product can be challenging since the interrelationship therebetween affects the product performance. New or improved features may interact unfavourably with other components and/or result in unacceptable performance trade-offs. For example, utilising a different propellant may result in an unacceptable droplet size of the ejected composition and consequently unsatisfactory hold, or a different container material may be incompatible with certain hairstyling polymers.

Nevertheless, sustainability, environmental and safety questions mean that there is a need for more environmentally friendly, more sustainable, safe and affordable hairspray products. Furthermore, there is a need for a hairspray product to be provided in a container with more flexibility as to shape and form. Furthermore, the container must be made from more sustainable materials and be easily recycled. However, these products must still exhibit excellent performance. Performance benefits may include, for example: excellent hold; long-lasting hold; good humidity resistance; shapeable hold; acceptable drying time; excellent soft, natural hair feel; acceptable or non-stickiness of the hands and hair. Of particular importance to the user is excellent hair hold.

### SUMMARY OF THE INVENTION

In a first aspect, the present invention relates to an aerosol hairspray product for styling and/or shaping hair wherein the product comprises:
i. a container for storing a hairstyling formulation and a propellant, wherein the container wall comprises at least about 80% plastic material by total weight of the container;
ii. a hairstyling formulation comprising:
   (a) from about 35% to about 99% water by total weight of the hairstyling formulation and propellant, and
   (b) from about 0.01% to about 10% hairstyling polymer by total weight of the hairstyling formulation and propellant;
iii. a propellant, which is selected from the group consisting of compressed gas propellants, liquefied gas propellants, and mixtures thereof;
iv. a spraying device;
   wherein the product comprises from 15% to 54% volatile organic compound by total weight of the hairstyling formulation and propellant;
   wherein the pressure inside the container is from about 1 to about 16 bar at 50°C.

In a second aspect, the invention relates to a method for styling hair comprising the steps of:
i. applying to hair a composition ejected by the hairspray product according to the present invention;
ii. drying the ejected composition on the hair.

In a third aspect, the invention relates to the use of the product according to the present invention for fixing and/or shaping a hairstyle.

### DETAILED DESCRIPTION OF THE INVENTION

In the first aspect, the present invention relates to an aerosol hairspray product. The use of said product results in the ejection of a sprayed composition, hereinafter the ejected composition, for application to human hair.

The term "aerosol" as used herein, means a suspension of fine droplets in a gas. The aerosol hairspray product atomises the hairspray formulation i.e. creates an aerosol. Due to surface tension, droplets are normally substantially spherical. As used herein, the "droplet size" is defined as the median diameter of ejected droplets.

The term "aerosol hairspray product" does not encompass mousse or foam products. The aerosol hairspray product herein is not a mousse or foam product. The term "mousse" or "foam" as defined herein means a dispersion of gas bubbles in a liquid. Commonly, mousse or foam compositions usually comprise greater than 0.3% surfactant by weight. The surfactant results in the formation of spherical bubbles which form the mousse or foam consistency. However, foams and mousses can also be formed from surfactant-free formulations via other means, for example special actuators, using proteins e.g. egg white protein. Typically, hairstyling products that eject a mousse/foam also comprise from about 6% to about 16% by weight of propellant.

The term "aerosol hairspray product" does not encompass gel products or products comprising or ejecting a gel composition. The aerosol hairspray product herein is not a gel product. Gels may be dispensed via a pump spray actuator. Both hand gel and spray gel formulations are not suitable for the present invention. Hand gel formulations typically have a viscosity of from about 8,000 mPa·s to about 20,000 mPa·s depending on the desired performance. Spray gels may have a lower viscosity, but the gel formulation can lead to clogging of the aerosol actuator insert. The ejected composition of spray gels typically has a droplet size of at least about 80 microns in diameter.

As used herein, the term "on-hair drying time" means the amount of time it takes for the ejected composition to dry on the hair. The on-hair drying time is measured by spraying a specific pattern on the hair and then timing when the hair ceases to feel tacky and damp in the hand.

As used herein, the term "ejection flow" is defined as the loss in total weight of the aerosol hairspray product after 5 seconds of spraying. This value is normally divided by 5 to give grams per sec. The ejection flow should achieve a balance between excellent hold and sufficiently fast drying time. For example, if too much ejected composition is applied to the hair in a short period, then the on-hair drying time may be unacceptably long.

Herein, "comprising" means that other steps and other ingredients which do not affect the end result can be added. This term encompasses the terms "consisting of" and "consisting essentially of". The compositions, methods, uses, and processes of the present invention can comprise, consist of, and consist essentially of the elements and limitations of the invention described herein, as well as any of the additional or optional ingredients, components, steps, or limitations described herein.

The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

The term "hairstyling polymer" as used herein means hair-fixing polymers which form films on a surface. In the context of hair, this surface is the surface of individual hair fibres or a plurality thereof. The polymer causes them to be glued together to build welds, which are crosslinks that provide the hold benefit. In concert, these welds form a 'hairnet' to provide hair hold and volume benefits to the user. When the net of welds is effectively formed, the hold and volume benefits can last all day and offer good resistance to environmental humidity.

The term "molecular weight" or "M.Wt." as used herein refers to the number average molecular weight unless otherwise stated.

All percentages are calculated by weight unless otherwise stated.

The hairspray product according to the present invention is suitable for application onto human hair. The term "suitable for application to human hair" as used herein means that the compositions or components thereof so described are suitable for use in contact with human hair and the scalp without undue toxicity, incompatibility, instability, allergic response, and the like.

The term "maximum incremental reactivity" value or "MIR" value as defined herein, means a measure of the increase in ozone formation per unit weight of a hydrocarbon when added to the atmosphere. Hence, MIR measured the ozone forming potential of a compound. A similar measurement to MIR is "photochemical ozone creation potential" or "POCP".

The term "global warming potential" or "GWP" as defined herein is a measure of how much a given mass of a compound is calculated to contribute to global warming compared to that of the same mass of carbon dioxide. The global warming potential of carbon dioxide, therefore, is 1. As used herein, the GWP values are those calculated for a 100 year time horizon, unless otherwise stated.

As used herein, the term "volatile organic compound" or "VOC", as used herein means any organic compound having a initial boiling point less than or equal to 250°C measured at a standard pressure of 101.3 kPa. Certain volatile compounds of organic chemistry falling within this definition are known to photochemically react with nitrogenic oxides in the presence of sunlight and, in turn, this produces ground-level ozone (O₃) and photochemical smog. Ground-level ozone can damage human health, damage vegetation. In fact, in the United States, the definition of VOC for US legislative purposes (U.S. EPA 40 CFR 51. 100[s]) defines only those organic compounds without negligible photochemical reactivity. Examples of compounds considered to be VOCs for the purposes of this application include: ethanol (EtOH), dimethylether (DME), 1,1-difluoroethane (HFC-152a), 1,1,1,2-tetrafluoroethane (HFC-134a), pentane, *n*-butane, *iso*-butane, propane, *trans*-1,3,3,3-tetrafluoropropene (HFO-1234ze), free formic acid (i.e. not its salt). Certain fragrances and plant extracts are also VOCs.

The term "non-flammable", as used herein in terms of the aerosol hairspray product, means the product contains 1% or less flammable components and the chemical heat of combustion is less than 20 kJ/g and is also considered non-flammable following an ignition distance test and, if necessary, the enclosed space test. If the chemical heat of combustion is less than 20 kJ/g, then the aerosol is classified as flammable if ignition occurs at a distance of 15 cm or more. The ignition distance test for spray aerosols is a standard test wherein the aerosol is sprayed in the direction of an ignition source at intervals of 15 cm to observe if ignition and sustained combustion takes place. Ignition and sustained combustion is defined as when a stable flame is maintained for at least 5 seconds. The ignition source is defined as a gas burner with a blue, non-luminous flame 4-5 cm in height. If no ignition occurs in the ignition distance test, the enclosed space test shall be performed and in this case, the aerosol is classified as flammable if the time equivalent is less than or equal to 300 s/m³ or the deflagration density is less than or equal to 300 g/m³; otherwise the aerosol is classified as non-flammable. The enclosed space ignition test is a standard test wherein the contents of an aerosol dispenser are sprayed into a cylindrical test vessel containing a burning candle. If an observable ignition occurs, the elapsed time and amount discharged is noted. These definitions are that of the UN Manual of Tests and Criteria, Part III, Section 31. The chemical heat of combustion can be determined via the standard method ASTM D 240.

The term "substantially free from", "substantially free of", or grammatical equivalents thereof, as defined herein means less than about 1%, preferably less than about 0.8%, more preferably less than about 0.5%, still more preferably less than about 0.3%, most preferably about 0%.

An aerosol hairspray product must offer to the user, in addition to excellent hairstyle hold, a variety of benefits. These benefits are delivered by the combination of container characteristics, spraying/ejection performance and also via the properties of the ejected hairspray composition on the hair. For example, the product must be easy to use - the container must be a suitable shape and size such that the user can easily hold it in one hand and, ideally with the same hand, be able to effect the spraying of the ejected composition, for example, by exerting pressure on a button. Therefore, the container must not slip in the user's hand. Furthermore, the user must be able to coat the targeted area of hair with the ejected composition - the user must have the ability to coat the entire hairdo with the ejected composition, or just a small section of the hairdo, as required. The expected styling benefit should be generated by the hairspray, for example firm hold, flexible hold, durable hold, shapeable hold etc.

Household containers, such as cosmetic product containers, are required to have certain physical properties to allow them to perform their intended function. Such items need to have mechanical properties such as sufficient mechanical strength to withstand, for example, the rigours of transport, storage and use. These rigours include e.g. stacking of items on top of each other (top-load), vibrations, shaking and other mechanical stresses, additionally temperature fluctuations during transportation, and usual handling stresses, such as being dropped and squeezed during consumer use. Thus, important mechanical properties include resistance to elongation, compression, flexion and temperature fluctuations. However, these containers must have, at the same time, a weight as low as possible in order to keep material consumption and the resulting environmental footprint, as well as transportation effort, low. In addition, such containers are also required to provide a high level of aesthetic appeal to consumers.

Currently, metal containers, e.g. made of tin plated steel or aluminium, are mostly used for containing aerosol hairsprays. Metal containers are strong and hence the container walls need only be comparatively thin. However, metal containers commonly need to be assembled together from different parts, for example, the walls, the base, the top. Also metal containers may have a higher explosion potential and often anti-corrosion measures are needed. Furthermore, metal containers feel cold to the touch and metal is a non-renewable resource.

Cosmetic products contained in plastic containers are commonly known. Plastic is a particularly advantageous material for containing cosmetic products because a greater variety of specific container forms may be created. The utilisation of plastic material(s) for a hairspray container provides an excellent means to deliver ease-of-use benefits to the consumer. For example, it is very easy to provide tactile advantages e.g. grip features, contours, and these tactile advantages can be designed with a high degree of specificity and accuracy. Furthermore, a plastic container can easily be moulded in one piece. Sealed plastic containers have a lower explosion potential than metal containers because, upon application of excessive temperature for example, due to the more elastic nature of plastic compared to metal, the plastic material can expand at a weak point in the container, e.g. where the container wall is thinner. Gradually and eventually the expansion at this weak point allows the high-pressured containers to escape via the formation of a hole. Furthermore, aesthetic benefits can also be realised more easily when a plastic container is used, for example, a transparent and/or translucent container material could be employed, and in addition to many other aesthetic benefits.

From an environmental perspective, utilisation of a plastic container has sustainability benefits and results in a reduced carbon footprint. Alternative, renewable, non-petroleum sources of oil can be utilised, for example, oil from plants. Ethylene, the monomer of polyethylene, may be made from sugarcane-derived ethanol. Furthermore, in recent years, significant advances have been made in the use of suitable sustainable filler materials to replace a proportion of the plastic material in order to answer sustainability questions. Plastic is also more easily recycled than metal.

However, hindrances exist in the use of plastic containers to contain aerosol products, which include both safety questions and physical questions. One such hindrance is the ability to retain an acceptable pressure inside the container. Firstly, compressed gas propellants are known to permeate through the plastic wall of the container. This can result in a gradual drop in container pressure over time. In the cosmetic field, this can be a significant issue because the product may be used relatively infrequently e.g. once per week, once per month. Therefore, the container may become unusable very quickly and while it still comprises a significant quantity of unused formulation. Moreover, the pressure inside of the container reduces each time the aerosol product is used. For aerosol products where compressed gas propellants, e.g. carbon dioxide, nitrogen, air, are used, this is a particular problem. The effect of this pressure drop and unacceptably low pressure inside the container is particularly pertinent to aerosol hairspray products where the physical properties of the ejected composition are of fundamental importance to the user. When the pressure inside the container is too low, an aerosol may not be created at all - the hairspray formulation is not released properly/optimally from the container, if at all. The performance of the hairspray may change dramatically from the point in time the product is new to when it is a few months old. For example, the product may first eject a formulation with a small droplet size, and then after a few months, a much larger droplet size - possibly even a stream or jet, which is totally unacceptable to a hairspray user. Alternatively, the product may, for example, first eject a highly dense or specific hotspot of ejected formulation, and then after a few months, a significantly less dense or different hotspot.

Another issue in relation to the use of plastic containers to contain aerosol products is the interaction of the propellant and hairstyling formulation with plastic material of the container. The utilisation of chemically organic propellants e.g. butane, in combination with a plastic container (also chemically organic) may result in thinning or pitting of the container wall due to chemical interactions between the propellant and the container. In such a situation, the container will not meet the physical requirements discussed above.

Furthermore, environmental and health questions exist for cosmetic products such as aerosol hairspray products. VOCs are compounds which, due to their significant vapour pressures, can easily escape from the cosmetic product into the atmosphere. The effect of this is two-fold: such chemicals may be breathed in and therefore may affect the health of living creatures; such chemicals are exposed to sunlight and therefore may participate in photochemical reactions in the atmosphere. Nitrogen oxides (NOₓ) can react with VOCs and cause ground-level ozone, which also has health impacts. Maximum incremental reactivity (MIR) is a measure of the ozone-forming potential of a compound and there are pressures to keep the calculated MIR value of products as low as possible. Therefore, there are ecological pressures to reduce, or avoid entirely, the use of VOCs in cosmetic products.

However, the high vapour pressure of VOCs has meant that they have historically been the preferred choice as the propellant for creating aerosols, such as in aerosol hairspray products. Other benefits of VOCs in the context of a hairspray product include: they can act as a solvent for the active ingredients, they help the product dry quickly, they help break the ejected composition into particles, and their high vapour pressure ensures a constant pressure in the container.

Another class of compounds with environmental questions includes chlorofluorocarbons (CFCs), which are chemical compounds comprising carbon, chlorine and fluorine atoms. When CFCs enter the atmosphere, they can react with UV light from the sun and form free radicals, which deplete stratospheric ozone, which, in turn, leads to climate change. CFCs have, therefore, a very high global warming potential (GWP). Alternatives to CFC propellants exist, for example 1,1-difluoroethane (also known as HFC-152a) and 1,1,1,2-tetrafluoroethane (HFC-134a), which do not comprise chlorine and therefore the scissile carbon-chlorine bond and consequently a much lower GWP. However, both HFC-152a and HFC-134a are VOCs.

Moreover, cosmetic products such as aerosol hairspray products must meet safety questions such as flammability. Aerosol hairspray products have conventionally comprised a high proportion of highly flammable compounds such as flammable propellants, hydrocarbons, and alcohols. In a fire, aerosol hairspray products can rupture and explode, and when comprising flammable compounds, may spread the fire further. Metal containers provide a substantial barrier between the formulation/propellant and an external fire source, and therefore a reduced fire hazard. Metal containers typically have a melting point of over 400°C and therefore are not subject to failure due to melting at relatively low fire temperatures (below 200°C). However, plastic containers typically have a melting point of less than 200°C and therefore represent more of a fire hazard than metal containers.

In addition, hairspray products comprising a high proportion of alcohol, often ethanol, are commonly used in the art. However, this high proportion of alcohol leaves the hair feeling dry and brittle. Also, ethanol may cause an allergic response in the user, is flammable and is a VOC.

The inventors have surprisingly overcome the above hindrances and answered the aforementioned needs by carefully selecting the specific combination of mutually compatible features such that the interaction therewith results in a hairspray with excellent performance. By utilising a plastic container, the user is provided with an environmentally friendly, easy to grip and precisely use hairspray product that feels warm to the touch and does not have the disadvantages of metal. The previous hindrances of utilising plastic container for aerosol hairspray products have been overcome by the utilisation of a high proportion of water and keeping the proportion of VOC, and MIR and GWP values very low, which answers the safety and environmental questions. The hairspray formulation, container material and wall thicknesses, and propellant type have been selected such that no incompatibility therebetween exists and excellent product performance results. Moreover, a aerosol hairspray product pursuant to the present invention avoids the drawbacks of using a high proportion of alcohol since it comprises a low proportion of alcohol, e.g. ethanol, if alcohol is present at all. A small proportion of surfactant is utilised where needed in order to ensure a suitable surface tension of the hairstyling formulation.

Performance benefits achieved by the hairspray product pursuant to the present invention include excellent hold, acceptable drying time, shapeable hold, excellent hair feel after brushing, feel after combing, and acceptable or non-stickiness of the hands and hair.

Each of the features of the aerosol hairspray product, as well as other relevant components, are described in detail hereinafter.

According to the first aspect, the present invention relates to an aerosol hairspray product comprising from 15% to 54% volatile organic compound by total weight of the hairstyling formulation and propellant. According to an embodiment, the product comprises from 15% to less than 30% volatile organic compound, by total weight of the hairstyling formulation and propellant. In another embodiment, the product comprises from 30% to less than 40% volatile organic compound, by total weight of the hairstyling formulation and propellant. In another embodiment, the product comprises from 40% to 54% of a volatile organic compound, by total weight of the hairstyling formulation and propellant.

According to an embodiment, the aerosol hairspray product has a maximum incremental reactivity (MIR) value of less than 1, more preferably less than 0.8, even more preferably less than 0.7. The MIR value of an aerosol hairspray product can be calculated by multiplying the fraction by weight of each component of the hairspray product by its MIR value. MIR values of common components of hairspray products include: 2-aminomethyl propanol has an MIR value of about 15.08; water has an MIR value of 0.00; acetone has an MIR of 0.43; ethanol has an MIR of 1.69; isopropanol has an MIR value of 0.71. More MIR values are listed in the propellants section. For example, a product comprising 0.2% of 2-aminomethyl propanol and no other components with an MIR value above zero, would have an MIR value of 0.03.

In an embodiment, the hairstyling formulation and propellant have a heat of combustion of from about 5 kJ/kg to about 20 kJ/kg and/or the product is non-flammable.

The aerosol hairspray product comprises a hairstyling formulation. The surface tension and viscosity of the hairstyling formulation is important because following spraying, the ejected composition forms droplets, which land on the hair. The ejected composition must then spread out along each individual hair fibre in order to form a thin layer of coating on the hair, which can dry quickly and also form welds with other similarly coated hair fibres.

In an embodiment of the present invention the surface tension, measured according to standard test ISO 304 at 20°C, of the hairstyling formulation is from about 20 mN/m to about 50 mN/m, preferably from about 20 mN/m to about 30 mN/m, more preferably from about 21 mN/m to about 25 mN/m. ISO 304 is a standard test method for measuring surface tension of pure liquids or solutions.

In an embodiment of the present invention, the kinematic viscosity, measured according to standard test DIN EN ISO 3104, of the hairstyling formulation is from about 1 to about 25 mm²/s, preferably from about 1 to about 15 mm²/s, more preferably from about 2 to about 11 mm²/s, most preferably from about 2 to about 8 mm²/s. When the hairspray product comprises from 40% to 54% of a volatile organic compound, by total weight of hairstyling formulation and propellant, the viscosity, measured according to standard test DIN EN ISO 3104, of the hairstyling formulation is from about 4 mm²/s to about 15 mm²/s. DIN EN ISO 3104 is a standard test method for measuring kinematic viscosity of liquids. The kinematic viscosity is important because when the hairstyling formulation is too viscous then the hairstyling is too thick and cannot be sprayed and/or is clogging - inhomogeneous ejected formulation may result e.g. irregular spray beam, "spitting" rather than spraying, and/or ejection of lumps. This is especially important when a compressed gas propellant is utilised because the propellant is in gas form and hence cannot function as a co-solvent.

The median droplet size of the ejected composition according to the present invention is from about 10 microns to about 80 microns, preferably from about 15 microns to about 50 microns, more preferably from about 15 microns to about 40 microns. Droplets smaller than about 10 microns are not suitable for the present invention due to safety concerns - the droplets may enter the lungs and cause health problems. Droplets larger than about 100 microns are too large and consequently unsuitable for the present invention. Hairspray products which are pump sprays normally have a droplet size which is too large and are hence unsuitable for the present invention. The aerosol hairspray product is not a pump spray.

Droplet size is measured using a technique based on laser diffraction. Scattered light is focused by a focusing lens in a Fourier arrangement and picked up by the detector array. The angle at which a particle diffracts light is inversely proportional to its size. The detector array is made up of over 30 individual detectors, each of which collects the light scattered by a particular range of angles. The scattering pattern from the spray is captured, which is what is measured. Measuring the angle of diffraction determines the size of the particle. A Malvern Spraytec EPCS 4.0 is used with a 450 mm lens type, serial number 237. Software: RT Sizer 5.0. Test duration: 4000 ms. Data acquisition rate: 200 Hz. Minimum particle size able to be measured: 0.8 µm. Maximum particle size able to be measured: 300 µm. Distance between nozzle and laser beam: 140 mm.

The ejection flow of the hairspray product is from about 0.10 g/sec to about 0.60 g/sec, preferably from about 0.20 g/sec to about 0.50 g/sec, most preferably from about 0.25 g/sec to about 0.35 g/sec. If the ejection flow is greater than about 0.60 g/sec, then the on-hair drying time will be too long for consumer satisfaction. Ejection flow can typically be adjusted by altering the pressure inside the container (increased pressure correlates with faster ejection flow) and/or the diameter opening in the spraying device and/or orifices in the actuator (lower diameter correlates with slower ejection flow).

The on-hair drying time of the ejected composition is from about 0.5 min to about 7 min, preferably from about 1 min to about 5 min, most preferably from about 1 min to about 2 min.

The hairstyling formulation comprises from about 0.01% to about 10% of a hairstyling polymer, preferably from about 1% to about 8% of a hairstyling polymer, more preferably from about 2% to about 6% of a hairstyling polymer, by total weight of the hairstyling formulation and propellant.

The hairstyling polymer is selected from the group consisting of amphoteric hairstyling polymers, zwitterionic hairstyling polymers, anionic hairstyling polymers, non-ionic hairstyling polymers, cationic hairstyling polymers, and mixtures thereof. In an embodiment, the hairstyling polymer is a water-compatible hairstyling polymer, alternatively a water-soluble hairstyling polymer. In an embodiment, the hairstyling formulation is substantially free from a water-incompatible hairstyling polymer. An example of a water-incompatible hairstyling polymer includes an Acrylates/t-Butylacrylamide Copolymer which is a copolymer of tert-butyl acrylamide and one or more monomers of acrylic acid, methacrylic acid, or one of their simple esters (e.g. Ultrahold^{®} 8 from BASF). Balance^{®} CR from Akzo Nobel, which is an acrylates copolymer of two or more monomers of (meth)acrylic acid or one of their simple esters, is however water-compatible and therefore is suitable for the present invention. The octylacrylamide/acrylate/butylaminoethyl methacrylate copolymer Amphomer^{®} is also water-compatible and therefore suitable. In an embodiment, the hairstyling polymer is a latex hairstyling polymer. In an embodiment, the product comprises less than about 0.5 wt% of a cationic hairstyling polymer by total weight of the hairstyling formulation and propellant.

The hairstyling formulation may comprise a cationic hairstyling polymer. Cationic hairstyling polymers suitable for the present invention may be selected from polymers with cationic or cationizable groups. Suitable cationic polymers include those with primary, secondary, tertiary or quaternary amino groups. The cationic charge density will be preferably from 1 to 7 meq/g. Suitable cationic polymers preferably contain quaternary amino groups. Cationic polymers can be homo- or copolymers, where the quaternary nitrogen groups are contained either in the polymer chain or preferably as substituents on one or more of the monomers. The ammonium group-containing monomers can be copolymerized with non-cationic monomers. Suitable cationic monomers are unsaturated compounds that can undergo radical polymerization, which bear at least one cationic group, especially ammonium-substituted vinyl monomers such as, for example, trialkylmethacryloxyalkylammonium, trialkylacryloxyalkylammonium, dialkyldiallylammonium and quaternary vinylammonium monomers with cyclic, cationic nitrogen-containing groups such as pyridinium, imidazolium or quaternary pyrrolidones, e.g. alkylvinylimidazolium, alkylvinylpyridinium, or alkylvinylpyrrolidone salts. The alkyl groups of these monomers are preferably lower alkyl groups such, as for example, C-1 to C-7 alkyl groups, and especially preferred are C-1 to C-3 alkyl groups.

The ammonium group-containing monomers can be copolymerized with non-cationic monomers. Suitable comonomers are, for example, acrylamide, methacrylamide, alkyl- and dialkylacrylamide, alkyl- and dialkylmethacrylamide, alkyl acrylate, alkyl methacrylate, vinylcaprolactone, vinylcaprolactam, vinylpyrrolidone, vinyl esters, for example vinyl acetate, vinyl alcohol, propylene glycol or ethylene glycol, where the alkyl groups of these monomers are preferably C-1 to C-7 alkyl groups, and especially preferred are C-1 to C-3 alkyl groups.

Suitable polymers with quaternary amino groups include, for example, those described in the CTFA Cosmetic Ingredient Dictionary under the designations 'polyquaternium' such as methylvinylimidazolium chloride/vinylpyrrolidone copolymer (polyquaternium-16) or quaternized vinylpyrrolidone/dimethylaminoethyl methacrylate copolymer (polyquaternium-11; Gafquat^{®} 755N-PW from ISP) as well as quaternary silicone polymers or silicone oligomers such as, for example, silicone polymers with quaternary end groups (quatemium-80).

Suitable cationic polymers of synthetic origin include: poly(dimethyldiallylammonium chloride); copolymers from acrylamide and dimethyldiallylammonium chloride; quaternary ammonium polymers, formed by the reaction of diethyl sulfate with a copolymer from vinylpyrrolidone and dimethylaminoethyl methacrylate, especially vinylpyrrolidone/dimethylaminoethyl methacrylate methosulfate copolymer (e.g. Gafquat® 755 N; Gafquat^{®} 734); quaternary ammonium polymers from methylvinylimidazolium chloride and vinylpyrrolidone (e.g. Luviquat^{®} HM 550 from BASF; Luviquat^{®} Hold from BASF; polyquaternium-46 [vinylcaprolactam {VCap}, vinylpyrrolidone {VP} and quaternized vinylimidazole {QVI}] from BASF; Luviquat^{®} FC 905 from BASF [polyquaternium-16]); Luviquat Supreme^{®} from BASF (polyquaternium-68, quaternised copolymer of vinyl pyrrolidone, methacrylamides, vinyl imidazole and quaternized vinyl imidazole); polyquaternium-35; polyquaternium-57; polymers from trimethylammonium ethyl methacrylate chloride; terpolymers from dimethyldiallylammonium chloride, sodium acrylate and acrylamide (e.g. Merquat® Plus 3300); copolymers from vinylpyrrolidone, dimethylaminopropyl methacrylamide, and methacryloylaminopropyllauryldimethylammonium chloride; terpolymers from vinylpyrrolidone, dimethylaminoethyl methacrylate, and vinylcaprolactam (e.g. Gaffix® VC 713); vinylpyrrolidone/methacrylamidopropyltrimethylammonium chloride copolymers (e.g. Gafquat® HS 100); copolymers from vinylpyrrolidone and dimethylaminoethyl methacrylate; copolymers from vinylpyrrolidone, vinylcaprolactam, and dimethylaminopropylacrylamide; poly- or oligoesters formed from at least one first type of monomer that is selected from hydroxyacids substituted with at least one quaternary ammonium group; dimethylpolysiloxane substituted with quaternary ammonium groups in the terminal positions.

Suitable cationic polymers that are derived from natural polymers include, in particular, cationic derivatives of polysaccharides, for example, cationic cellulose derivatives, starch, or guar. Cationic polysaccharides are, for example, represented by the general formula:

G-O-B-N⁺-R^{a}-R^{b}-R^{c} X⁻

G is an anhydroglucose residue, for example, starch or cellulose anhydroglucoses;
B is a divalent bonding group, for example, alkylene, oxyalkylene, polyoxyalkylene or hydroxyalkylene;
R^{a}, R^{b} and R^{c} are, independently from one another, alkyl, aryl, alkylaryl, arylalkyl, alkoxyalkyl or alkoxyaryl, any of which can have up to 22 carbon atoms, wherein the total number of carbon atoms in R^{a}, R^{b} and R^{c} is preferably a maximum of 20;

Cationic cellulose derivatives include those that have at least one quaternary ammonium group, e.g. a copolymer made of hydroxyethyl cellulose and diallyldimethyl ammonium chloride (polyquaternium-4), or the reaction product made of hydroxyethyl cellulose and an epoxide substituted with a trialkyl ammonium group (polyquaternium-10), wherein the alkyl groups can have 1 to 20 carbon atoms, and methyl groups are preferred. The molecular weight is preferably between 100,000 and 600,000, but 200,000 to 400,000 is especially preferred. The nitrogen content is preferably 0.5 to 4%, with 1.5 to 3% being especially preferred. The preferred cellulose derivative is polyquaternium-4, which is sold under the trade names Celquat® H1OO and Celquat^{®} L200, of which Celquat^{®} L200 is especially preferred.

Cationic latex hairstyling polymers are also suitable.

The preferred cationic polymers are polyquaternium-4, polyquaternium-11, polyquaternium-16, polyquaternium-68, mixtures thereof, and mixtures of polyquaternium-68 with non-ionic polymers. The most preferred cationic polymers are polyquaternium-4, polyquaternium-11, polyquaternium-68, and mixtures thereof.

In an embodiment, the hairstyling formulation comprises less than 0.1% by weight chitosan, chitosan salts and chitosan derivatives. In another embodiment, the hairstyling formulation is substantially free from chitosan, chitosan salts and chitosan derivatives.

When the aerosol hairspray product comprises from 15% to less than 30% VOC, preferred cationic polymers are polyquaternium-4, polyquaternium-11, polyquaternium-16, polyquaternium-68, mixtures thereof; and the most preferred are polyquaternium-4, polyquaternium-68, and mixtures thereof.

When the aerosol hairspray product comprises from 30% to less than 54% VOC, preferred cationic hairstyling polymers are polyquaternium-4, polyquaternium-11, polyquaternium-68, mixtures thereof; and the most preferred are polyquaternium-4, polyquaternium-68, and mixtures thereof.

The hairstyling formulation may comprise amphoteric or zwitterionic hairstyling polymers. Zwitterionic and amphoteric polymers may be selected from: copolymers formed from alkylacrylamide, alkylaminoalkyl methacrylate, and two or more monomers from acrylic acid and methacrylic acid as well as, if necessary, their esters, especially copolymers from octylacrylamide, acrylic acid, butylaminoethyl methacrylate, methyl methacrylate and hydroxypropyl methacrylate (octylacrylamide/acrylate/butylaminoethyl methacrylate copolymer, for example Amphomer^{®} from Akzo Nobel); copolymers, that are formed from at least one of a first type of monomer that possesses quaternary amino groups and at least one of a second type of monomer that possesses acid groups; .copolymers from fatty alcohol acrylates, alkylamine oxide methacrylate and at least one monomer selected from acrylic acid and methacrylic acid as well as, if necessary, acrylic acid esters and methacrylic acid esters, especially copolymers from lauryl acrylate, stearyl acrylate, ethylamine oxide methacrylate and at least one monomer selected from acrylic acid and methacrylic acid as well as, if necessary, their esters; copolymers from methacryloyl ethyl betaine and at least one monomer selected from methacrylic acid and methacrylic acid esters; copolymers from acrylic acid, methyl acrylate and methacrylamidopropyltrimethylammonium chloride (polyquaternium-47); copolymers from acrylamidopropyltrimethylammonium chloride and acrylates or copolymers from acrylamide, acrylamidopropyltrimethylammonium chloride, 2-amidopropylacrylamide sulfonate and dimethylaminopropylamine (polyquaternium-43); oligomers or polymers, producible from quaternary crotonoylbetaines or quaternary crotonoylbetaine esters.

Amphoteric and zwitterionic latex hairstyling polymers are also suitable.

Amphoteric polymers such as Amphomer^{®} are preferably present in their neutralized or partially neutralized form. Suitable neutralisers include potassium hydroxide, sodium hydroxide, triisopropanolamine (TIPA), 2-aminobutanol, 2-aminomethyl propanol (AMP), aminoethylpropandiol, dimethyl stearamine (Armeen 18 D), sodium silicate, tetrahydroxypropyl ethylenediamine (Neutrol^{®} TE), ammonia (NH₃), triethanolamine, trimethylamine (Tris Amino Ultra), aminomethylpropandiol (AMPD), preferably 2-aminobutanol, ammonia, and 2-aminomethyl propanol. A particularly preferred neutralising agent for Amphomer^{®} is 2-aminomethyl propanol.

The preferred amphoteric or zwitterionic polymers are polyquaternium-47, octylacrylamide/acrylate/butylaminoethyl methacrylate copolymers, and mixtures thereof; most preferably octylacrylamide/acrylate/butylaminoethyl methacrylate copolymers, particularly Amphomer^{®} from Akzo Nobel.

The hairstyling formulation may comprise anionic polymers. Suitable anionic hairstyling polymers are selected from among: acrylates copolymers of two or more monomers of (meth)acrylic acid or one of their simple esters (e.g. Balance^{®} CR from Akzo Nobel); acrylates/hydroxyesters acrylates copolymers including those being copolymers of butyl acrylate, methyl methacrylate, methacrylic acid, ethyl acrylate and hydroxyethyl methacrylate (e.g. Acudyne™ 1000 from Dow Personal Care); terpolymers of acrylic acid, ethyl acrylate, and N-tert-butylacrylamide; crosslinked or uncrosslinked vinyl acetate/crotonic acid copolymers; terpolymers of tert-butylacrylate, ethyl acrylate and methacrylic acid; sodium polystyrenesulfonate; copolymers of vinyl acetate, crotonic acid and vinyl propionate; copolymers of vinyl acetate, crotonic acid and vinyl neodecanoate; aminomethylpropanol/acrylate copolymers; copolymers of vinylpyrrolidone and at least one further monomer selected from among acrylic acid, methacrylic acid, acrylic acid esters and methacrylic acid esters; copolymers of methyl vinyl ether and maleic acid monoalkyl esters; aminomethylpropanol salts of copolymers of allyl methacrylate and at least one further monomer selected from among acrylic acid, methacrylic acid, acrylic acid esters and methacrylic acid esters; crosslinked copolymers of ethyl acrylate and methacrylic acid; copolymers of vinyl acetate, mono-n-butyl maleate and isobornyl acrylate; copolymers of two or more monomers selected from among acrylic acid, methacrylic acid, acrylic acid esters and methacrylic acid esters, copolymers of octylacrylamide and at least one monomer selected from among acrylic acid, methacrylic acid, acrylic acid esters and methacrylic acid esters; polyesters of diglycol, cyclohexanedimethanol, isophthalic acid and sulfoisophthalic acid; polyurethanes; and copolymers of polyurethane and acrylates. e.g. polyurethane-14/AMP-acrylates polymer blend (e.g. DynamX^{®} from Akzo Nobel). Suitable polyester polymers include polyester-5 polymers, for example AQ^{®} 48 Ultra Polymer, (diglycol/CHDM/isophthalates/SIP copolymer [a copolymer of diethylene glycol, 1,4-cyclohexanedimethanol and the simple esters of isophthalic acid and sulfoisophthalic acid]), AQ^{®} 55 S, and AQ^{®} 38 S, all from Eastman Chemical Company. Also suitable is a polyvinylmethacrylic acid/maleic acid copolymer (Omnirez^{®} 2000 from ISP). Anionic latex hairstyling polymers are also suitable.

The preferred anionic polymers are selected from the group consisting of polyurethane-1 (e.g. Luviset^{®} P.U.R. from BASF), polyurethane-14/AMP-acrylates copolymer blend (e.g. DynamX^{®} from Akzo Nobel), acrylates copolymers of two or more monomers of (meth)acrylic acid or one of their simple esters (e.g. Balance^{®} CR from Akzo Nobel), and mixtures thereof. The most preferred anionic polymer is polyurethane-1.

When the aerosol hairspray product comprises from 41% to 54% VOC, the preferred anionic polymers are selected from the group consisting of polyurethane-14/AMP-acrylates copolymer blend (e.g. DynamX^{®} from Akzo Nobel), polyester-5 polymers, (e.g. AQ^{®} 48 Ultra Polymer, AQ^{®} 55 S, AQ^{®} 38 S, all from Eastman Chemical Company), and mixtures thereof.

Anionic polymers are preferably present in their neutralized or partially neutralized form. Suitable neutralisers include potassium hydroxide, sodium hydroxide, triisopropanolamine (TIPA), 2-aminobutanol, 2-aminomethyl propanol (AMP), aminoethylpropandiol, dimethyl stearamine (Armeen 18 D), sodium silicate, tetrahydroxypropyl ethylenediamine (Neutrol^{®} TE), ammonia (NH₃), triethanolamine, trimethylamine (Tris Amino Ultra), aminomethylpropandiol (AMPD), preferably 2-aminobutanol, ammonia, and 2-aminomethyl propanol. A particularly preferred neutralising agent is 2-aminomethyl propanol.

The hairstyling formulation may comprise a non-ionic hairstyling polymer. Suitable non-ionic polymers are homo- or copolymers that are formed from at least one of the following monomers: vinylpyrrolidone, vinylcaprolactam, vinyl esters such as, for example, vinyl acetate, vinyl alcohol, acrylamide, methacrylamide, alkyl- and dialkylacrylamide, alkyl- and dialkylmethacrylamide, alkyl acrylate, alkyl methacrylate, propylene glycol or ethylene glycol, where the alkyl groups in these monomers are preferably C-1 to C-7 alkyl groups, and C-1 to C-3 alkyl groups are especially preferred. Suitable homopolymers are, for example, those of vinylcaprolactam, vinylpyrrolidone or N-vinylformamide. Further suitable non-ionic hairstyling polymers include copolymers of vinylpyrrolidone and vinyl acetate, terpolymers of vinylpyrrolidone, vinyl acetate and vinyl propionate, polyacrylamides; polyvinyl alcohols as well as polyethylene glycol/polypropylene glycol copolymers. Also suitable are polyvinylpyrrolidone/dimethylaminopropylaminoacrylates copolymer (Aquaflex^{®} SF 40 from ISP); isobutylene ethylmaleinimide/hydroxy ethylmaleinimide copolymer (Aquaflex^{®} FX 64 from ISP); vinylcaprolactam/polyvinylpyrrolidone/dimethylaminoethyl methacrylate copolymer (Advantage^{®} from ISP). Non-ionic latex hairstyling polymers are also suitable.

Preferred non-ionic polymers include polyvinylpyrrolidone (PVP); polyvinylpyrrolidone/ vinylacetate (PVP/VA) copolymers; polyvinylpyrrolidone, polyvinylcaprolactam, vinylpyrrolidone/vinyl acetate copolymers; copolymers of vinylpyrrolidone, methacrylamide and vinylimidazole; polyvinyl alcohol, isobutylene/ethylmaleimide/hydroxyethylmaleimide copolymers; and copolymers of vinylpyrrolidone, vinyl acetate and vinyl propionate. More preferred non-ionic polymers include polyvinylpyrrolidone (K90, 85, 80, 60, 30), polyvinylpyrrolidone/vinyl acetate copolymers (PVP/VA 64), terpolymers of vinylpyrrolidone, methacrylamide and vinylimidazole (e.g. Luviset^{®} Clear from BASF), and mixtures thereof. The even more preferred non-ionic polymers include PVP K 60, 30, and PVP/VA 37/64. The most preferred non-ionic polymers include PVP K60 and PVP/VA 37/64.

When the aerosol hairspray product comprises from 40% to 54% VOC, preferred the preferred non-ionic polymers are: polyvinylpyrrolidone polymers, more preferably vinylpyrrolidone/vinyl acetate copolymers (PVP/VA), most preferably PVP/VA 37.

The hairstyling formulation may comprise latex hairstyling polymers. As used herein, a "latex hairstyling polymer solution" is a droplet of latex hairstyling polymer in water. The advantage of latex polymer solutions is that they have a similar viscosity to water, even when the composition comprises up to 50% by weight latex polymer. Water thin polymer solutions can be sprayed easily without clogging the valve-insert combination and still provide good hold.

Latex hairstyling polymers are selected from the group consisting of anionic latex hairstyling polymers, amphoteric latex hairstyling polymers, non-ionic latex hairstyling polymers, cationic latex hairstyling polymers, and mixtures thereof.

Suitable anionic latex polymers include urethane-based polymers, for example polyurethane-34 (Baycusan® from Bayer). Polyurethane-34 is described in EP2105127A1. In an embodiment of the present invention, the hairstyling polymer is the latex hairstyling polymer polyurethane-34. Suitable amphoteric latex hairstyling polymers include latex resins based on styrene/butylacrylate or methylmethacrylate/butylacrylate latex mixed with Amphomer^{®} and AMP (EP0688557B1). Suitable non-ionic latex hairstyling polymers include styrene-butadiene polymers, acrylic, vinyl acetate polymers, and mixtures thereof, with non-ionic ethylene oxide/propylene oxide block copolymer surfactants (US 5,525,657). Suitable cationic latex hairstyling polymers include cationic graft-modified rubber latex polymers with a cationic and/or non-ionic surfactant (US6512034).

The hairstyling formulation may comprise blends of hairstyling polymers. When there is a blend of a cationic hairstyling polymer combined with an anionic hairstyling polymer then the cationic hairstyling polymer is preferably less than about 0.2%, more preferably less than about 0.15%, even more preferably less than about 0.1%, by weight of the hairstyling formulation and propellant. Suitable polymer combinations include the following: cationic cellulose derivatives of hydroxyethyl cellulose and diallyldimethylammonium chloride in conjunction with vinylpyrrolidone/vinyl acetate copolymers; chitosan in conjunction with polyvinylpyrrolidone; quaternary ammonium polymers of methylvinylimidazolium chloride and vinylpyrrolidone in conjunction with vinylpyrrolidone/vinyl acetate copolymers and/or polyvinylpyrrolidone; and octylacrylamide/acrylates/butylaminoethyl methacrylate copolymer (Amphomer®) with polyvinylpyrrolidone (PVP).

The two most preferred polymer combinations are: vinylpyrrolidone/vinyl acetate copolymers (PVP/VA), and polyvinylpyrrolidone (PVP); and octylacrylamide/acrylates/butylaminoethyl methacrylate copolymer (Amphomer®) with polyvinylpyrrolidone (PVP).

The hairstyling formulation may further include a surfactant. The hairstyling formulation may comprise 1% or less surfactant, preferably 0.6% or less, more preferably 0.4% or less, even more preferably 0.3% or less, by total weight of the hairstyling formulation and propellant. The surfactant is selected from the group consisting of cationic surfactants, non-ionic surfactants, anionic surfactants, and mixtures thereof.

Cationic surfactants are selected from the group consisting of cetrimonium chloride (e.g. Quartamin 60L-G from Kao; DEHYQUART A-CA /DETEX; ARQUAD 16-25 LO); cocamidopropyl hydroxysultaine (e.g. REWOTERIC AM CAS); cocamidopropyl betaine (e.g. TEGO BETAIN F 50); betaine; and mixtures thereof.

Non-ionic surfactants are selected from the group consisting of castor oil PEG-40 H (e.g. NEODOL 91-8); laureth-4 (e.g. DEHYDOL LS 4 DEO N); laureth-9; decyl glucoside (e.g. Plantacare 2000); polysorbate 20 (e.g. TWEEN 20 PHARMA from UNIQEMA); PEG-25 hydrogenated castor oil (e.g. SIMULSOL 1292 DF from SEPPIC); PEG-40 hydrogenated castor oil (e.g. CREMOPHOR CO 410 from BASF); PPG-1-PEG-9-laurylglycolether (e.g. Eumulgin L); siloxane polyalkyleneoxide copolymer (Silwet^{®} L7604 from Momentive); and polydimethylsiloxane methylethoxylate (Silwet^{®} L7600 from Momentive); and mixtures thereof.

A suitable anionic surfactant is dioctyl sodium sulfosuccinate (DOSS or 1,4-dioctoxy-1,4-dioxobutane-2-sulfonic acid), an example of which is Aerosol OT-70 PG from Cytec.

Preferred surfactants include: castor oil PEG-40 H; cetrimonium chloride; laureth-4; laureth-9; decyl glucoside; cocamidopropyl hydroxysultaine; polysorbate 20; siloxane polyalkyleneoxide copolymer; dioctyl sodium sulfosuccinate; and mixtures thereof. More preferred surfactants include: castor oil PEG-40 H; decyl glucoside; cocamidopropyl hydroxysultaine; polysorbate 20; siloxane polyalkyleneoxide copolymer; dioctyl sodium sulfosuccinate; and mixtures thereof. The most preferred surfactants include: siloxane polyalkyleneoxide copolymer; and dioctyl sodium sulfosuccinate; and mixtures thereof.

The hairstyling formulation comprises from about 35% to about 99% water, preferably from about 40% to about 99%, more preferably from about 50% to about 99%, even more preferably from 60% to about 99%, by total weight of the hairstyling formulation and propellant.

Alcohol may be present in the aerosol hairspray product. Suitable alcohols for inclusion in the hairstyling formulation are selected from the group consisting of ethanol, isopropanol, and mixtures thereof. Ethanol and/or isopropanol may be added to the hairspray product in order to assist the drying of the ejected hairspray composition on the hair. Ethanol is more preferred than isopropanol. Both ethanol and isopropanol are flammable and VOCs. In an embodiment, the product comprises a maximum of 54% alcohol by total weight of the hairspray formulation and propellant.

The hairstyling formulation may further comprise any component suitable for incorporation in an aerosol hairspray formulation.

The hairstyling formulation may comprise at least one preservative, preferably less than 1.5% preservative, more preferably 0% to 1% by total weight of the hairstyling formulation and propellant. Suitable preservatives include: phenoxyethanol (Euxyl^{®} PE 9010), propyleneglycol, octylsalicylate, 1,3-bis(hydroxymethyl)-5,5-dimethylimidazolidine-2,4-dione (DMDM hydantoin; Nipaguard^{®} DMDMH by Clariant), EDTA (Rexat), butylene glycol (Dekaben LMB), and parben types e.g. methylparaben (e.g. PHB-methyl ester from Schütz & Co., or SLI CHEMICALS, or Nipagin^{®} M), propylparaben (PHB-PROPYLESTER from SOLVADIS SPECIALTIES).

The present invention may further comprise at least one perfume or fragrance. The aerosol hairspray product may comprise a maximum of about 0.5% perfume or fragrance, preferably from about 0% to about 0.4%, more preferably from about 0.1% to about 0.3%, by total weight of the hairstyling formulation and propellant.

The aerosol hairspray product may further comprise vitamins and amino acids such as: water soluble vitamins such as vitamin B1, B2, B6, B12, C, pantothenic acid, pantothenyl ethyl ether, panthenol, biotin, and their derivatives, water soluble amino acids such as asparagine, alanine, indole, glutamic acid and their salts, water insoluble vitamins such as vitamin A, D, E, and their salts and/or derivatives, water insoluble amino acids such as tyrosine, tryptamine, viscosity modifiers, dyes, non-volatile solvents or diluents (water soluble and insoluble), pearlescent aids, foam boosters, additional surfactants or non-ionic cosurfactants, pediculocides, pH adjusting agents, perfumes, preservatives, chelants, proteins, skin active agents, sunscreens, UV absorbers, vitamins, niacinamide, caffeine and minoxidil. The product may comprise from about 0% to about 5% vitamins and amino acids, by total weight of the hairstyling formulation and propellant.

The aerosol hairspray product may further comprise pigment materials such as inorganic pigments, nitroso-, monoazo-, disazo-compounds, carotenoid, triphenyl methane, triaryl methane, chemicals of the quinoline, oxazine, azine, or anthraquinone type, as well as compounds which are indigoid, thionindigoid, quinacridone, phthalocianine, botanical, natural colors, and preferably water-soluble components. The product may comprise from about 0% to about 5% pigment materials, by total weight of the hairstyling formulation and propellant. The compositions of the present invention may also contain antimicrobial agents which are useful as cosmetic biocides. The product may comprise from about 0% to about 5% antimicrobial agents, by total weight of the hairstyling formulation and propellant.

The hairstyling formulation has a pH of from about 6 to about 10, preferably from about 7 to about 10, more preferably from about 7 to about 9.The product comprises a propellant, which is selected from the group consisting of compressed gas propellants, liquefied gas propellants, and mixtures thereof.

The compressed gas propellants are selected from the group consisting of air, nitrogen (N₂), nitrous oxide (N₂O), carbon dioxide (CO₂), and mixtures thereof; preferably air, nitrogen (N₂), and mixtures thereof; most preferably nitrogen (N₂). In an embodiment, the compressed gas propellant is not carbon dioxide (CO₂) - particularly when a hairstyling polymer may precipitate due to effect of the CO₂ in lowering the pH of the hairstyling formulation. Also CO₂ typically permeates through plastic material to a greater or lesser extent i.e. 0% permeation is typically unachievable. The term "air" is defined herein as a gas comprising approximately 78% nitrogen, 21% oxygen, and 1% of carbon dioxide, argon and other trace elements. Since the content of air can vary, the compressed gas propellant is preferably nitrogen gas. As defined herein, the compressed gases N₂, CO₂, and N₂O are all non-flammable. N₂O has a GWP of 298. When the propellant is air, a maximum of 1 g is utilised as propellant.

The liquefied gas propellants are selected from the group consisting of dimethylether (DME), 1,1-difluoroethane (HFC-152a), 1,1,1,2-tetrafluoroethane (HFC-134a), pentane, *n-*butane, *iso*-butane, propane, *trans*-1,3,3,3-tetrafluoropropene (HFO-1234ze), and mixtures thereof, preferably dimethylether (DME), 1,1-difluoroethane (HFC-152a), and mixtures thereof.

For the purposes of the present invention, all the liquefied gas propellants mentioned above are VOCs. Furthermore, as defined herein, *n*-butane is flammable, has an MIR of 1.15 and has a GWP of 4; *iso*-butane is flammable and has an MIR of 1.23; propane is flammable, has a GWP of 3.3 and an MIR of 0.49; HFC-134a is non-flammable, has a GWP of about 1400, MIR of 0.00; HFC-152a is flammable, has a GWP of about 120, MIR of 0.02; HFO-1234ze is non-flammable, has a GWP of 6, MIR of 0.09; DME is flammable, has a GWP of 1, and has a MIR of 0.81.

The aerosol hairspray product comprises from 15% to 54% VOC by total weight of the hairstyling formulation and propellant. When the liquefied gas propellant is a VOC and the product comprises no other VOCs, the product comprises from 15% to 54% liquefied gas propellant, by total weight of the hairstyling formulation and propellant. When other VOCs are used in the aerosol hairspray product, for example ethanol, then less liquefied gas propellant may be used.

CFCs are not suitable propellants for the present invention due to their ozone depleting properties. For example, CFC-12 has a GWP of 10,900. In an embodiment, the product has a GWP of 100 or less, preferably 50 or less, more preferably 20 or less, even more preferably 10 or less, most preferably 5 or less.

According to the first aspect, the present invention relates to an aerosol hairspray product for styling and/or shaping hair wherein the product comprises a container for storing a hairstyling formulation and a propellant, wherein the container wall comprises at least about 80% plastic material by total weight of the container. Pressurizable containers for holding and dispensing consumer products, such as shaving cream, air fresheners, cleaners, furniture polish, etc are well known in the art.

The term "plastic" is defined herein as any polymeric material that is capable of being shaped or molded, with or without the application of heat, and then hardened into a desired form including, polymers, resins, and cellulose derivatives. Usually plastics are homo- or co-polymers of high molecular weight.

In an embodiment of the present invention, the plastic material is selected from the group consisting of polyolefins, polyesters, polyamide, polyvinylchloride, acrylic, polycarbonates, polyethylene naphthalate (PEN), polyethylene therephthalate (PET), polystyrene, polyurethane, and mixtures thereof; more preferably polyethylene terephthalate (PET), polyethylene napththalate (PEN), and mixtures thereof.

The hairspray product according to the present invention comprises a container wherein container wall comprises at least about 80% plastic material, preferably from about 85% to about 100% by total weight of the container.

Plastics can be defined by their glass transition temperature (Tg) and/or molecular weight. The wall thickness of the plastic container is also important. For an example PET container, a glass transition temperature of 75°C is used and a container with a wall thickness from about 0.5 mm to about 3.2 mm is used. An example PET container comprises the following wall thicknesses: shoulder about 0.65 mm; sidewall about 0.50 mm; outside base about 1.09 mm; base pushup about 2.90 mm.

The container pursuant to the present invention can be pressurised to a pressure greater than atmospheric pressure using propellants, inflatable bags, powered pumps, and manual pumps such as a squeeze trigger. The pressure inside the container can be measured with a pressure gauge (GCAS #60001439). The container must be able to withstand a pressure inside the container of about 16 bar at 50 °C. According to the first aspect, the pressure inside the container is from about 1 bar to about 16 bar at 50°C. When the propellant is a compressed gas, the pressure inside the container is from about 6 bar to about 12 bar, preferably from about 8 bar to about 10 bar, most preferably about 9 bar, at 50°C. When the propellant is a liquefied gas, the pressure inside the container is from about 1 bar to about 7 bar, preferably from about 1.5 bar to about 5 bar, at 50°C. When the propellant is *iso*-butane, propane, or dimethylether the pressure inside the container is preferably from about 3 bar to about 4 bar, at 50°C. When the propellant is *n*-butane, the pressure inside the container is preferably from about 1.5 bar to about 2 bar, most preferably 1.7 bar to about 1.9 bar, at 50°C.

According to an embodiment, the product comprises, in addition to the container, a nozzle, valve, and sealing mounting cup. US 3,819,090 relates to a valve cup device for pressurized dispensing containers comprising a one-piece molded plastic body. US 5,199,615 A relates to an aerosol dispenser.

The sealing valve and actuator may or may not be made from plastic. Valve, actuator and inserts are available from Seaquist Closures (Freyung, Germany), Precision and Coster (Switzerland).

In an embodiment, the product comprises a variable spray-angle nozzle, variable resin flux nozzle, and/or helix atomizer nozzle. In an embodiment, the container comprises a maximum volume of 220 ml of hairstyling formulation and propellant.

The container may be moulded to create a specific ergonomic external form or contour, for example, hand-shaped contours. Said form facilitates effective and precise use of the hairspray product, for example by providing more grip or non-slip. Other tactile features may also be provided on the surface of the container, for example pimples. Furthermore the container may be provided with specific aesthetic features, such as colour combinations, and transparent or translucent portions. In an embodiment, at least 50 wt% of the container wall is translucent, more preferably transparent. When externally viewable, bag-on-valve systems are less favoured by consumers for aesthetic reasons. In an embodiment, the aerosol hairspray product does not comprise a bag-on-valve system when a portion of the container wall is translucent, preferably transparent.

Other polymers that may be utilized in the container of the present invention include polymers made from components derived from renewable sources i.e. non-petroleum sources. As used herein the term "sustainable polymer" means polymers made from components e.g. monomers, derived from renewable sources. Examples of renewable, non-petroleum sources include plants and microorganisms. The renewable, non-petroleum plants sources may include sugar cane, beets, corn, potatoes, citrus fruit, and woody plants. For example, ethanol can be produced from sugarcane. The ethanol may then be converted into ethylene, which can be polymerized to form polyethylene (PE). The monomers from which polypropylene (PP), polyester, and polyethylene terephthalate (PET) are synthesized, may also be derived from renewable sources. Sustainable polymers may be synthesized from monomers derived from starch and/or cellulose, or by modification of the polymer itself. Cellulosics are thermoplastic resins manufactured by the chemical modification of cellulose.

These sustainable plastic materials may be used as 100% of the plastic utilized for the container of the present invention, or blended into the petroleum-derived plastic at varying levels in order to vary performance and/or for economic reasons.

Certain materials derived from plant sources may be biodegradable. Sustainable polymers exhibiting biodegradability include aliphatic polyesters such as polylactic acid (PLA), polyglycolic acid (PGA), polybutylene succinate (PBS) and copolymers thereof, aliphatic-aromatic polyesters such as Ecoflex® from BASF and Biomax® from DuPont, polyhydroxyalkanoate (PHA) and copolymers thereof. Thermoplastic starch (TPS) materials are also biodegradable, as are cellulosics. The incorporation of biodegradable sustainable polymers may be at 100% of the utilized plastic or in blends with other materials, in order to control the speed or degree of biodegradation, or for economic reasons. The speed and degree of biodegradation must be compatible with the purpose and features of the present invention. Ecoflex® from BASF, for example, is a biodegradable plastic material that biodegrades in soil or compost. It is stable on shelf for one year. It is particularly suitable for bags and films.

Recycled plastic can also be re-ground. This post-consumer regrind resin may also be suitable for the present invention either when blended with other resins or used as 100% of the plastic utilised. Re-ground polyethylene at certain densities (r-HDPE, r-LLDPE, r-LDPE), reground polypropylene (r-PP), and reground polyethylene terephthalate (r-PET) may be suitable.

Filler materials may be blended into the plastic utilized for the present invention. The advantages of the incorporation of filler materials in plastic include: adjustment of physical properties of the plastic, such as mechanical strength, density and cooling time, and also economic reasons. Suitable fillers may include starches, fibres from renewable sources such as hemp, flax, coconut, wood, paper, bamboo, and also inorganic materials such as calcium carbonate, mica, and talc. In addition, gas fillers such as high pressure gas, foaming agents or microspheres may be added to the plastic of the present invention.

In a particularly preferred embodiment, the present invention relates to an aerosol hairspray product for styling and/or shaping hair wherein the product comprises:
i. a container for storing a hairstyling formulation and a propellant, wherein the container wall comprises at least about 85% polyethylene terephthalate by total weight of the container;
ii. a hairstyling formulation comprising:
   (a) from about 35% to about 99% water by total weight of the hairstyling formulation and propellant, and
   (b) from about 0.01 % to about 10% hairstyling polymer by total weight of the hairstyling formulation and propellant, wherein the hairstyling polymer is selected from the group consisting of octylacrylamide/acrylates/butylaminoethyl methacrylate copolymers, polyquaternium-11 polymers, vinylpyrrolidone/vinylacetate copolymers, and mixtures thereof, and
   (c) an alcohol selected from the group consisting of ethanol, isopropanol, and mixtures thereof;
iii. a propellant, which is dimethylether;
iv. a spraying device;
wherein the product comprises from 40% to 54% VOC volatile organic compound by total weight of the hairstyling formulation and propellant;
wherein the pressure inside the container is from about 1 bar to about 16 bar at 50°C.

In a second aspect, the invention relates to a method for styling hair comprising the steps of: (i) applying to hair an ejected composition, which is ejected by the hairspray product according to the present invention; (ii) drying the ejected composition on the hair. The method may also comprise a step preceding step (i) wherein a hairdo or hairstyle is created. The method may also comprise a step preceding step (ii) but after step (i) wherein a hairdo or hairstyle is created.

Step (i), the application step, may comprise the applying the ejected composition to sections of hair or to an entire head of hair. Step (ii), the drying step, may consist of drying shaped treated hair from min at a temperature above room temperature. Step (ii) is conducted after step (i), preferably immediately afterwards or at least from about 1 to about 60 min after step (i). Alternatively, step (ii) may be conducted using any suitable device such as a blow dryer, a hood, curling tongs, or straightening irons. Alternatively or complementarily, step (ii) may be conducted merely by leaving hair to dry naturally at room temperature for a period of time, preferably less than 30 min. The on-hair drying time of the ejected composition is from about 0.5 min to about 7 min, preferably from about 1 min to about 5 min, most preferably from about 1 min to about 2 min.

Step (i) may be preceded by a step or steps selected from the group consisting of a stylist consultation step, shampooing step, a conditioning step, a hair treatment step, a hair cutting step, a hair colouring step, a hair finishing step, and a hair styling step. Step (i) may also be preceded by a step comprising chemically modifying the internal region of a hair shaft.

In a third aspect, the invention relates to the use of the product according to the present invention for fixing and/or shaping a hairstyle.

In an embodiment of the third aspect, the use comprises using the product according to the present invention for fixing a hairstyle following the creation of a hairstyle.

Alternatively, the use comprises using the product according to the present invention for creating and shaping a hairstyle.

The present invention may further comprise an article of commerce comprising at least one aerosol hairspray product, as described herein, and a communication pertaining to the product. The communication may be printed material attached directly or indirectly to packaging containing at least one aerosol hairspray product pursuant to the present invention. Alternatively, the communication may be an electronic or a broadcast message that is associated with a hairstyling device and/or the at least one aerosol hairspray product. The communication may comprise images comparing the appearance of a person prior to use of the product to the appearance of the same person after using the product.

The present invention may further comprise a kit comprising at least one aerosol hairspray product, as described herein, and at least one communication, as described herein. The kit may further comprise an item selected from the group consisting of a shampoo product, conditioner product, mousse product, gel product, a hairstyling tool, blow dryer, curling tongs, and straightening irons. The hairstyling tool may be selected from the group consisting of hair bands, hair fasteners, combs, and brushes.

### METHOD OF MAKING A HAIRSPRAY

First two solutions are made: a main mix and a second mix. The main mix comprises the hairstyling polymer(s), which are dissolved with stirring in water and components of the preservative system. A second mix is created which comprises water and the paraben-based preservative component(s) (e.g. methyl paraben). The second mix is normally heated up in a microwave to 90 to 95°C in order to dissolve the paraben. The two mixes are then combined to create the hairstyling formulation. The hairstyling formulation is then put into the container and then container is sealed by crimping on a sealing mounting cup. Then the propellant is added under pressure and then the valve system and nozzle is added to the container.

### EXAMPLES

### Examples 1 - 5: Comprising from 15% to less than 30% VOC

| | Ex. 1 | Ex. 2 | Ex. 3 | Ex. 4 | Ex. 5 |
|---|---|---|---|---|---|
| Vinylpyrrolidone/vinylacetate copolymer¹ | 3.0 | - | - | 2.0 | - |
| Polyvinylpyrrolidone ² | - | 3.0 | - | - | 3.0 |
| Polyurethane-14/AMP acrylates polymer blend | - | - | 0.5 | 0.25 | 0.1 |
| 2-aminomethyl propanol (AMP) | - | - | 0.29 | 0.15 | 0.09 |
| Castor oil PEG-40 H, (90%) | 0.3 | 0.3 | 0.3 | 0.35 | 0.35 |
| Disodium EDTA | - | - | - | 0.15 | 0.15 |
| Perfume | 0.2 | 0.2 | 0.2 | 0.1 | 0.1 |
| Phenoxyethanol ⁴ | 0.7 | 0.7 | 0.7 | 0.4 | 0.4 |
| 1,3-bis(hydroxymethyl)-5,5-dimethylimidazolidine-2,4-dione ⁵ | 0.4 | 0.4 | 0.4 | - | - |
| Methylparaben ⁶ | - | - | - | 0.2 | 0.2 |
| Ethanol | 10 | 12 | 20 | 25 | 29 |
| Deionised water | Add to 100 | Add to 100 | Add to 100 | Add to 100 | Add to 100 |

| | | | | | |
|---|---|---|---|---|---|
| **Key:** ¹ = Luviskol^{®} VA 64; ² = Luviskol^{®} K30; ³ = DynamX^{®} from Akzo Nobel; ⁴ = Euxyl^{®} PE 9010; ⁵ = Nipaguard^{®} DMDMH; ⁶ = PHB-methylester from Schütz. | | | | | |

An exemplified hairstyling formulation from one of examples 1 to 5 is placed in a suitable plastic container. The propellant is a compressed gas e.g. air, nitrogen, or a liquefied gas such as dimethylether, equipped with a suitable spraying device, such that the product comprises from 15% to less than 30% VOC.

### Examples 6 - 15: Comprising from 30% to 54% VOC

| | Ex. 6 | Ex. 7 | Ex. 8 | Ex. 9 | Ex. 10 |
|---|---|---|---|---|---|
| Vinylpyrrolidone/vinylacetate copolymer¹ | 5.0 | 5.0 | 3.0 | 3.0 | 3.0 |
| Polyvinylpyrrolidone ⁷ | - | 5.0 | 2.0 | 1.0 | 2.0 |
| Polyquaternium-11 ⁸ | - | - | - | 0.2 | 0.15 |
| Castor oil PEG-40 H, (90%) | 0.35 | 0.35 | 0.35 | 0.4 | 0.4 |
| Disodium EDTA | - | - | - | 0.13 | 0.13 |
| Perfume | 0.2 | 0.2 | 0.2 | 0.1 | 0.1 |
| Phenoxyethanol ⁴ | 0.7 | 0.7 | 0.7 | 0.4 | 0.4 |
| 1,3-bis(hydroxymethyl)-5,5-dimethylimidazolidine-2,4-dione ⁵ | 0.4 | 0.4 | 0.4 | - | - |
| Methylparaben ⁶ | - | - | - | 0.2 | 0.2 |
| Ethanol | 15 | 24 | 34 | 44 | 54 |
| Deionised water | Add to 100 | Add to 100 | Add to 100 | Add to 100 | Add to 100 |

| | | | | | |
|---|---|---|---|---|---|
| **Key:** as above, and ⁷ = Luviskol^{®} K60; ⁸ = Gafquat^{®} 755N-PW from ISP. | | | | | |

| | Ex. 11 | Ex. 12 | Ex. 13 | Ex. 14 | Ex. 15 |
|---|---|---|---|---|---|
| Vinylpyrrolidone/vinylacetate copolymer ¹ | 3.0 | - | 4.0 | 2.0 | 3.5 |
| Polyvinylpyrrolidone ² | - | 3.0 | 1.0 | 2.0 | 1.5 |
| Polyester-5 polymer ⁹ | 0.2 | 0.1 | 0.15 | 0.3 | 0.25 |
| Dioctyl sulfosuccinate ¹⁰ | - | 0.15 | - | - | 0.1 |
| Disodium EDTA | - | - | - | 0.13 | 0.13 |
| Perfume | 0.2 | 0.2 | 0.2 | 0.1 | 0.1 |
| Phenoxyethanol ⁴ | 0.7 | 0.7 | 0.7 | 0.4 | 0.4 |
| 1,3-bis(hydroxymethyl)-5,5-dimethylimidazolidine-2,4-dione ⁵ | 0.4 | 0.4 | 0.4 | - | - |
| Methylparaben ⁶ | - | - | - | 0.2 | 0.2 |
| Ethanol | 20 | 25 | 35 | 45 | 54 |
| Deionised water | Add to 100 | Add to 100 | Add to 100 | Add to 100 | Add to 100 |

| | | | | | |
|---|---|---|---|---|---|
| **Key:** as above, and ⁹ = AQ^{®} 48 Ultra Polymer from Eastman Chemical Company; ¹⁰ = Aerosol OT-70 PG from Cytec. | | | | | |

An exemplified hairstyling formulation from one of examples 6 to 15 is placed in a suitable plastic container. The propellant is a compressed gas e.g. air, nitrogen, or a liquefied gas such as dimethylether, equipped with a suitable spraying device, such that the product comprises from 30% to 54% VOC.

### Examples 16-20: Comprising from 40% to 54% VOC

| | Ex. 16 | Ex. 17 | Ex. 18 | Ex. 19 | Ex. 20 |
|---|---|---|---|---|---|
| Vinylpyrrolidone/vinylacetate copolymer ¹ | 3.0 | - | - | 2.0 | - |
| Polyquaternium-11 ⁸ | - | 3.0 | - | - | 1.5 |
| Octylacrylamide/acrylate/butylamin oethyl methacrylate copolymer 11 | 0.5 | 1.0 | 2.5 | 2.0 | 2.0 |
| 2-aminomethyl propanol (AMP) | 0.12 | 0.24 | 0.48 | 0.4 | 0.4 |
| Castor Oil PEG-40 H, (90%) | 0.35 | 0.35 | 0.35 | 0.4 | 0.4 |
| PEG-90 hydrogenated castor oil | - | - | - | 0.2 | 0.2 |
| Disodium EDTA | - | - | - | 0.13 | 0.13 |
| Perfume | 0.2 | 0.2 | 0.2 | 0.1 | 0.1 |
| Phenoxyethanol ⁴ | 0.7 | 0.7 | 0.7 | 0.4 | 0.4 |
| 1,3-bis(hydroxymethyl)-5,5-dimethylimidazolidine-2,4-dione ⁵ | 0.4 | 0.4 | 0.4 | - | - |
| Methylparaben ⁶ | - | - | - | 0.2 | 0.2 |
| Ethanol | 10.0 | 10.0 | 15.0 | 25.0 | 30.0 |
| Deionised water | Add to 100 | Add to 100 | Add to 100 | Add to 100 | Add to 100 |

| | | | | | |
|---|---|---|---|---|---|
| **Key:** as above, and ¹¹ = Amphomer^{®} from Akzo Nobel. | | | | | |

An exemplified hairstyling formulation from one of examples 16 to 20 is placed in a suitable plastic container. The propellant is a compressed gas e.g. air, nitrogen, or a liquefied gas such as dimethylether, equipped with a suitable spraying device, such that the product comprises from 40% to 54% VOC.

### HAIRSPRAY PERFORMANCE

The performance of the aerosol hairspray product pursuant to the present invention may be tested using various techniques. These test methods include: measurement of the on-hair drying time of the ejected composition; analysis of the spreading behaviour of the ejected composition on the hair via microscopy, for example; an analysis wherein the density of ejected composition over a specific area is measured and concentration core is identified; sensory analysis of the feel of the hair and hold provided by the ejected composition by a panel of specialists; and sensory analysis of the performance of the container, spraying means, nozzle etc also by a panel of specialists.

### PERFORMANCE TEST

A group of 10 consumers were asked a series of questions in relation to a product pursuant to the present invention. The consumers were regular hairspray users. The consumers were internal employees of the Procter & Gamble Company, but were not informed as to whether the products shown were competitor's products or Procter & Gamble products, nor the purpose of the testing. None of the employees were working in the patent department. The product was an aerosol hairspray wherein the container wall was made from PET; the hairstyling formulation comprised 7.5 wt% (by total weight of the hairstyling formulation and propellant) of AQ™ 48 as hairstyling polymer, 73.14 wt% (by total weight of the hairstyling formulation and propellant) of water, and 20 wt% (by total weight of the hairstyling formulation and propellant) of ethanol; nitrogen gas was the propellant; the pressure inside the container was 6.8 bar at room temperature.

Each consumer used the product by spraying one half side of a mannequin head and was subsequently asked whether they agreed or disagreed with specific performance criteria. The results are shown in Table A below.

**TABLE A**

| | Consumer (Base= 10) | |
|---|---|---|
| Criteria: | AGREE | DON'T AGREE |
| Spray properties- sprays like a hairspray | 8 | 2 |
| Hair feel - felt like a hairspray (on mannequin head) | 9 | 1 |
| Hold- delivers hold like a hairspray | 9 | 1 |
| Meets expectation of an hairspray | 8 | 2 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An aerosol hairspray product for styling and/or shaping hair wherein the product comprises:
i. a container for storing a hairstyling formulation and a propellant, wherein the container wall comprises at least about 80% plastic material by total weight of the container;
ii. a hairstyling formulation comprising:
(a) from about 35% to about 99% water by total weight of the hairstyling formulation and propellant, and
(b) from about 0.01% to about 10% hairstyling polymer by total weight of the hairstyling formulation and propellant, and
iii. a propellant, which is selected from the group consisting of compressed gas propellants, liquefied gas propellants, and mixtures thereof;
iv. a spraying device;
wherein the product comprises from 15% to 54% VOC volatile organic compound by total weight of the hairstyling formulation and propellant;
wherein the pressure inside the container is from about 1 bar to about 16 bar at 50°C.

2. The product according to claim 1, further comprising an alcohol selected from the group consisting of ethanol, isopropanol, and mixtures thereof.

3. The product according to any preceding claim, wherein the compressed gas propellants are selected from the group consisting of air, nitrogen, nitrous oxide, carbon dioxide, and mixtures thereof; preferably air, nitrogen, and mixtures thereof; most preferably nitrogen.

4. The product according to any preceding claim, wherein the liquefied gas propellants are selected from the group consisting of dimethylether, 1,1-difluoroethane, 1,1,1,2-tetrafluoroethane, pentane, *n*-butane, *iso*-butane, propane, *trans*-1,3,3,3-tetrafluoropropene, and mixtures thereof; preferably dimethylether, 1,1-difluoroethane, and mixtures thereof.

5. The product according to any preceding claim, wherein the hairstyling formulation comprises 1% or less surfactant, preferably 0.6% or less, more preferably 0.4% or less, even more preferably 0.3% or less, by total weight of the hairstyling formulation and propellant.

6. The product according to any preceding claim, wherein the kinematic viscosity, measured according to standard test DIN EN ISO 3104, of the hairstyling formulation is from about 1 to about 25 mm²/s, preferably from about 1 to about 15 mm²/s, more preferably from about 2 to about 11 mm²/s, most preferably from about 2 to about 8 mm²/s.

7. The product according to any preceding claim, comprising from about 1% to about 8% of a hairstyling polymer, preferably from about 2% to about 6% of a hairstyling polymer, by total weight of the hairstyling formulation and propellant.

8. The product according to any preceding claim, wherein the plastic material is selected from the group consisting of polyolefins, polyesters, polyamide, polyvinylchloride, acrylic, polycarbonates, polyethylene naphthalate, polyethylene terephthalate, polystyrene, polyurethane, and mixtures thereof, more preferably polyethylene terephthalate, polyethylene napththalate, and mixtures thereof.

9. The product according to any preceding claim, wherein the hairstyling formulation comprises a surfactant selected from the group consisting of cationic surfactants, non-ionic surfactants, anionic surfactants, and mixtures thereof.

10. The hairspray product, according to any preceding claim, which comprises from 40% to 54% of a volatile organic compound, by total weight of the hairstyling formulation and propellant.

11. The hairspray product, according to claim 10, wherein the propellant is a liquefied gas and the pressure inside the container is from about 1 bar to about 7 bar, preferably from about 1.5 bar to about 5 bar, at 50°C.

12. The hairspray product, according to claim 10 or claim 11, wherein the hairstyling polymer is selected from the group consisting of octylacrylamide/acrylates/butylaminoethyl methacrylate copolymers, polyquaternium-11 polymers, vinylpyrrolidone/vinylacetate copolymers, and mixtures thereof.

13. The product according to any preceding claim, wherein the surface tension, measured according to standard test ISO 304 at 20°C, of the hairstyling formulation is from about 20 mN/m to about 50 mN/m, preferably from about 20 mN/m to about 30 mN/m, more preferably from about 21 mN/m to about 25 mN/m.

14. A method for styling hair comprising the steps of:
i. applying to hair an ejected composition, which is ejected by the hairspray product according to any of the preceding claims;
ii. drying the ejected composition on the hair.

15. The use of the product according to any of claims 1 to 13, for fixing and/or shaping a hairstyle.
